(19) Europäisches Patentamt
European Patent Office
Office européen des brevets

(11) Numéro de publication: **0 135 489**
**B1**

(12) # FASCICULE DE BREVET EUROPÉEN

(45) Date de publication du fascicule du brevet: **04.11.87**

(51) Int. Cl.⁴: **C 07 D 307/80**, A 61 K 31/34

(21) Numéro de dépôt: **84870102.5**

(22) Date de dépôt: **12.07.84**

(54) **Nouveaux dérivés de benzofuranne, leur préparation et leur utilisation thérapeutique.**

(30) Priorité: **18.07.83 FR 8311805**

(43) Date de publication de la demande: **27.03.85 Bulletin 85/13**

(45) Mention de la délivrance du brevet: **04.11.87 Bulletin 87/45**

(84) Etats contractants désignés: **AT CH DE FR GB IT LI LU NL SE**

(56) Documents cité:
**FR-A-2 242 087**

**EXPERIENTIA, vol. 31, no. 11, 15 novembre 1975, pages 1322-1323, Bâle, CH; G.A. FOTHERGILL et al.: "Bufuralol, a new beta-adrenoceptor blocking agent"**

**Le dossier contient des informations techniques présentées postérieurement au dépôt de la demande et ne figurant pas dans le présent fascicule.**

(73) Titulaire: **SANOFI, société anonyme, 40, Avenue George V, F-75008 Paris (FR)**

(72) Inventeur: **Descamps, Marcel, Rue du Bois du Bosquet, 30, B-1331 Rosieres (BE)**
Inventeur: **Polster, Peter, Rue Champ d'Oiseaux, 9bis, B-5990 Beauvechain (Hamme- Mille) (BE)**

(74) Mandataire: **Cauchie, Daniel, c/o S.A. LABAZ-SANOFI N.V. Avenue De Béjar, 1, B-1120 Bruxelles (BE)**

**EP 0 135 489 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention se rapporte, d'une manière générale, à des dérivés hétérocycliques et plus particulièrement à de nouveaux dérivés de benzofuranne ainsi qu'à leur procédé de préparation.

Les dérivés de benzofuranne de la présente invention peuvent être représentés par la formule générale:

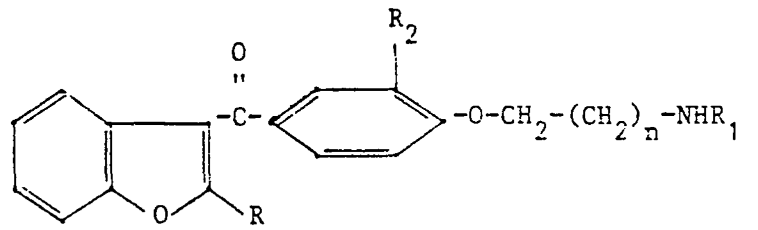

I

dans laquelle:

R représente un radical éthyle n-propyle isopropyle ou n-butyle

$R_1$ représente un radical alkyle ramifié ayant de 4 à 7 atomes de carbone dans lequel au moins un des atomes de carbone ne porte pas d'hydrogène, par préférence un radical tertiobutyle ou néopentule

$R_2$ représente hydrogène, chlore ou brome

n représente 1 ou 2.

L'invention se rapporte également aux sels d'addition non toxiques des composés de formule I, par exemple le chlorhydrate.

On a trouvé que les dérivés de benzofuranne de l'invention possèdent de remarquables propriétés pharmacologiques notamment des propriétés inhibitrices de la translocation calcique ainsi que des propriétés antiadrénergiques. Ces propriétés sont capables de rendre les composés en question très utiles dans le traitement de certains syndromes pathologiques du système cardiovasculaire, en particulier dans le traitement de l'angine de poitrine, de l'hypertension, de l'arythmie, de l'insuffisance circulatoire cérébrale. Dans le domaine antitumoral, les composés de l'invention pourront être utiles comme potentialisateurs d'anticancéreux.

En conséquence, l'invention se rapporte également à des compositions pharmaceutiques ou vétérinaires contenant, comme principe actif, au moins un dérivé de benzofuranne de formule I ou un sel d'addition non toxique de ce dérivé en association avec un véhicule pharmaceutique ou un excipient approprié. Selon la voie d'administration choisie, la posologie journalière pour un être humain pesant 60 kg se situera entre 2 et 500 mg de principe actif. Les composés de formule I peuvent être préparés, selon l'invention, en condensant, dans un milieu inerte tel que le benzène, le toluène, l'éthanol ou un mélange de ces solvants, un composé benzofurannique de formule générale:

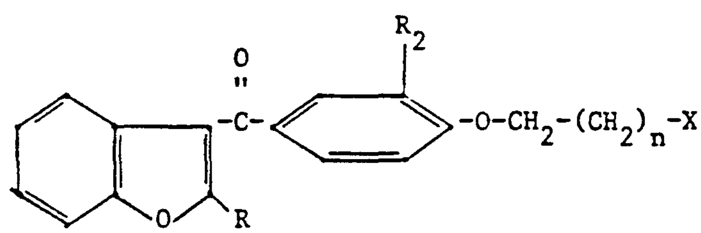

II

dans laquelle R et $R_2$ ont la même signification que précédemment et X représente un atome d'halogène de préférence le brome ou un groupement p-toluènesulfonyloxy, avec une amine de formule générale:

$H_2N-R_1$ III

dans laquelle $R_1$ a la même signification que dans la formule I, pour former le dérivé souhaité de benzofuranne de formule I que l'on fait réagir, si on le désire, avec un acide organique ou inorganique approprié pour obtenir un sel d'addition non toxique.

La condensation en question sera effectuée à la température ambiante ou à une température comprise entre la température ambiante et 80° C.

Les composés de formule II peuvent être obtenus:

a) lorsque X est un halogène, par condensation d'un dérivé de benzofuranne de formule:

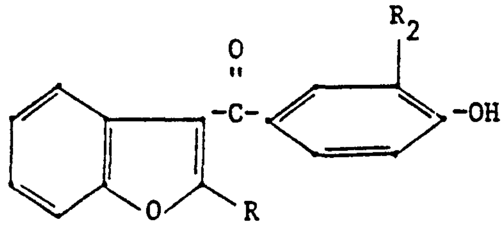

IV

dans laquelle R et $R_2$ ont la même signification que dans la formule I, avec un dihalogénoalkane de formule générale:

$Hal\text{-}CH_2\text{-}(CH_2)_n\text{-}Hal$ V

dans laquelle Hal représente un atome d'halogène de préférence le brome et n a la même signification que dans la formule I, et ce dans un solvant tel que la méthyl éthyl cétone ou le diméthylformamide et en présence d'un carbonate de métal alcalin par exemple le carbonate de potassium

b) lorsque X est un groupement p-toluènesulfonyloxy, par condensation du chlorure de p-toluènesulfonyle dans la pyridine, avec un dérivé de benzofuranne de formule générale:

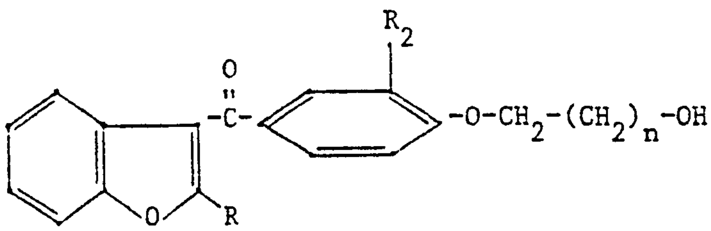

VI

dans laquelle R, $R_2$ et n ont la même signification que dans la formule I. Quant aux composés de formule VI, ceux-ci peuvent être préparés en condensant dans un solvant tel que le diméthylformamide et en présence de carbonetes de métal alcalin par exemple le carbonate de potassium, un dérivé de benzofuranne de formule IV ci-dessus avec un alcool halogéné de formule générale:

$Hal\text{-}CH_2\text{-}(CH_2)_n\text{-}OH$ VII

dans laquelle Hal et n ont la même signification que dans la formule V ci-dessus.

Suivant une autre méthode, on prépare les composés de formule II ci-dessus dans laquelle X représente un halogène, en condensant, selon la réaction de FRIEDIL et CRAFTS, un chlorure d'acyle de formule:

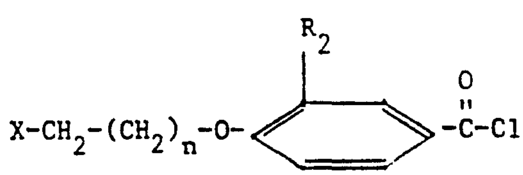

VIII

dans laquelle $R_2$ et n ont la même signification que dans la formule I et X représente un atome d'halogène de préférence le brome, avec un dérivé de benzofuranne de formule générale:

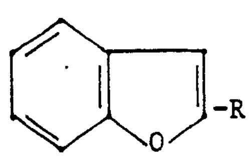

IX

dans laquelle R a la même signification que dans la formule I.

Les composés de formule IX sont tous des produits connus d'après le brevet français No. 1.260.578 tandis que ceux de formule IV sont également des composés connus ou pouvant être préparés selon des méthodes décrites dans le susdit brevet français.

On connaît déjà des dérivés de benzofuranne substitués par une chaîne monoalkylaminoalkyloxybenzoyle et

présentés comme ayant des effets pharmacologiques capables de les rendre utiles dans le traitement de l'angine de poitrine. A cet effet, on peut citer le brevet français No. 2.242.087 lequel décrit spécifiquement les n-butyl-2[(méthylamino-3 ou éthylamino-3 ou n-butylamino-3 propoxy)-4 benzoyl]-3 benzofurannes ainsi que l'éthyl-2(diméthyl-3,5 γ-isopropylaminopropoxy-4 benzofuranne, ces composés étant sous forme de leur chlorhydrate.

Toutefois, le brevet en question ne contient aucun résultat de test pharmacologique pour les dérivés monoalkyles.

D'autre part, le n-butyl-2[(éthylamino-2 éthoxy)-4 diiodo-3,5 benzoyl]-3 benzofuranne a été mentionné dans Hellenic Biochemical and Biophysical Society Newletter June 1981, No. 16 pages 6-8 sans qu'aucune activité pharmacologique n'y ait été citée.

En plus, des essais effectués dans le cadre de la présente invention ont révélé, pour ces composés connus, des potentialités antiadrénergiques nulles ou faibles, en tout état de cause, trop faibles pour être d'un apport quelconque à la thérapeutique.

On a découvert de manière surprenante, dans le cadre de la présente invention, qu'en remplaçant le radical monoalkyle au niveau de la chaîne monoalkylaminoalkoxybenzoyle des composés connus par un radical monoalkyle ramifié dans lequel au moins un des atomes de carbone ne porte pas d'hydrogène notamment le radical tertiobutyle ou néopentyle, on obtient des composés présentant des potentialités antiadrénergiques α et β beaucoup plus importantes que chez les composés de l'art antérieur. D'autre part, on a constaté que l'activité inhibitrice calcique des composés de l'invention est au moins égale sinon supérieure à celle observée lors de tests pratiqués avec les composés connus. Au contraire des composés connus, on a ainsi pu mettre en évidence pour les composés de la présente invention, un spectre pharmacologique faisant apparaître les composantes anticalcique et antiadrénergique α et β avec un équilibre d'intensité thérapeutiquement valable par exemple pour le traitement de l'angor.

Au surplus, les composés de l'invention sont dépourvus d'atomes d'iode contrairement au dérivé éthylamino-benzofuranne mentionné dans Hellenic Biochemical and Biophysical Society Newletter ci-dessus et à l'amiodarone ou n-butyl-2[(diéthylamino-2 éthoxy)-4 diiodo-3,5-benzoyl]-3 benzofuranne, un composé largement reconnu pour ses propriétés antiangineuses et antiarythmiques. Or, il est souhaitable de disposer d'autres composés actifs dépourvus d'iode dans leurs molécules. En effet, l'administration régulière de substances comportant ce halogène peut conduire à des effets secondaires indésirables particulièrement au niveau de la glande thyroide. En outre, de telles substances peuvenr rendre extrêmement difficile tout examen précis de la thyroïde. Finalement, les composés de l'invention, de par leur faible toxicité, pourraient être utilisés comme médicaments.

Comme il a été rapporté en détail par R. CHARLIER dans "Bruxelles Médical" n° 9, septembre 1969, pages 543-564, il est admis qu'une médication antiangineuse doit être capable notamment d'antagoniser les réactions cardiovasculaires de type adrénergique.

A cet effet, on a proposé des agents aptes à bloquer les récepteurs α. Cependant, l'application clinique de tels composés au traitement de l'angor resta sans succès très probablement par le fait que les antagonistes des récepteurs α n'induisent qu'une neutralisation très partielle ou système adrénergique, l'activité des récepteurs β étant respectée.

Or, les manifestations hémodynamiques les plus indésirables qui surviennent chez l'angineux de poitrine au cours de ses accès douloureux, sont avant tout cardiaques et relèvent de ce fait des récepteurs β.

Parallèlement, on a proposé des médications antagonistes des récepteurs adrénergiques β. Ces composés, d'un intérêt clinique réel, diminuent les crises d'angor en réduisant le travail cardiaque par ralentissement de la fréquence du rythme. Cependant, il n'y a pas de chute des résistances artérielles périphériques qui s'élèvent au contraire par libération du tonus α. Ces médications modifient toutefois certains paramètres hémodynamiques dans un sens qui, sur le plan fondamental, constitue une contrepartie défavorable à l'angineux de poitrine en particulier et au cardiaque en général. Si l'on considère l'aspect antiadrénergique des β-bloquants, il devient évident que seules la tachycardie et l'augmentation de la puissance et de la vélocité de la contraction cardiaque sont susceptibles d'être neutralisées, l'hypertension artérielle relevant d'une stimulation des récepteurs α sur lesquels les antagonistes β n'ont pas d'impact.

Or, si les perturbations cardiovasculaires entraînées par la stimulation des récepteurs β sont les plus préjudiciables à l'angineux, il n'en reste pas moins que l'hypertension artérielle joue également un rôle qui n'est pas négligeable.

Au surplus, le blocage des récepteurs β comporte un risque privant l'insuffisant cardiaque d'un mécanisme compensateur qu'il met normalement en jeu pour limiter son insuffisance circulatoire.

Ce mécanisme réflexe dont la composante principale emprunte la voie du système β-adrénergique aboutit notamment à une augmentation de la puissance et de la vélocité de la contraction cardiaque. Par conséquent, si ce système est bloqué, l'insuffisant cardiaque voit s'aggraver sa défaillance fonctionnelle. Il est donc logique de considérer que l'emploi d'un β-bloquant dont l'action est pure et complète comportera toujours un risque cardiaque. Il paraît donc souhaitable de ne pas rechercher des propriétés antagonistes α ou β complètes, étant donné les effets secondaires qu'elles peuvent entraîner en clinique. Il semble plus logique de viser à amortir plutôt qu'à supprimer les perturbations cardiovasculaires qui caractérisent l'hyperstimulation du système adrénergique dans sa totalité.

Les composés de l'invention répondent à cet objectif puisqu'ils présentent des propriétés antiadrénergiques de type α et β incomplètes. Ils peuvent donc être considérés, non comme des β-bloquants mais comme des

adrénofreinateurs c'est-à-dire des antagonistes partiels des réactions adrénergiques α et β potentiellement dépourvus des désavantages énumérés ci-dessus pour les β-bloquants.

En outre, la composante inhibitrice calcique mise en évidence chez les composés de l'invention complètera d'une manière remarquable leur spectre pharmacologique cardiovasculaire.

On sait que les ions calcium agissant au niveau cellulaire contrôlent le degré de vasoconstriction et, par la même occasion, jouent un rôle critique dans la crise d'angine de poitrine.

Les composés antagonistes du calcium agissent au niveau de la membrane cellulaire en empêchant sélectivement le calcium d'intervenir dans le processus de contraction au sein de la cellule artérielle.

Or, il apparaît de plus en plus évident, à l'heure actuelle, que les résultats cliniques apportés par l'association d'inhibiteurs calciques et d'inhibiteurs β-adrénergiques sont meilleurs que lorsque chaque inhibiteur est utilisé isolément (J.A.M.A. 1982, 247, pages 1911-1917).

Il semble, en outre, qu'il n'existe, à l'heure actuelle, aucun β-bloquant exerçant en plus une action inhibitrice appréciable au niveau de la translocation calcique.

De ce point de vue, les composés de l'invention présentant à la fois une composante anticalcique et une composante antiadrénergique α et β seront d'un intérêt primordial car susceptibles d'applications thérapeutiques plus étendues qu'un β-bloquant isolé ou qu'un inhibiteur calcique isolé. A titre d'exemple, on citera le n-butyl-2[(néopentylamino-2 éthoxy)-4 benzoyl]-3 benzofuranne qui présente une composante antiadrénergique α et β doublée d'un effet économisateur d'oxygène pouvant apporter un effet thérapeutique chez l'homme dans le syndrome d'angine d'effort, lequel peut par ailleurs être traité par les β-bloquants classiques. Cependant, l'intérêt majeur de ce composé résidera dans le fait qu'il pourra, grâce à sa composante anticalcique, être utilisé dans le traitement de l'angine au repos, syndrome provoqué par l'apparition d'un spasme au niveau des coronaires lequel est combattu actuellement par des composés tels que le diltiazem, le vérapamil ou la nifédipine. Les résultats de tests pharmacologiques effectués en vue de déterminer les propriétés cardiovasculaires des composés de l'invention sont répertoriés ci-dessous.

**I. Propriétés inhibitrices calciques**

Les propriétés inhibitrices de la translocation calcique au niveau membranaire présentées par les composés de l'invention ont été mises en évidence par mesure de leur action antagoniste vis-à-vis de la réponse contractile à la dépolarisation provoquée par le potassium sur l'aorte isolée de rat. Il est bien établi que la dépolarisation de la membrane d'un muscle lisse par le potassium rend cette dernière perméable au calcium extracellulaire et provoque la contraction musculaire.

Dès lors, la mesure de l'inhibition de la réponse contractile à la dépolarisation par le potassium ou la mesure d'un relâchement de la contraction tonique à la dépolarisation potassique peut constituer une évaluation de la puissance d'un composé en tant qu'inhibiteur de la perméabilité membranaire aux ions $Ca^{++}$.

La technique utilisée est la suivante:

Sur des rats mâles Wistar pesant environ 300 g, on prélève l'aorte et on la coupe en bandelettes d'environ 40 mm de longueur et 3 mm de largeur. On place ces fragments dans une cuve à organe isolé de 25 ml contenant une solution de Krebs-bicarbonate modifiée (NaCl 112 m M; KCl 5 m M; $NaHCO_3$ 25 m M; $KH_2PO_4$ 1 m M; $MgSO_4$ 1,2 m M; CaCl 2,5 m M; glucose 11,5 m M; eau distillée jusqu'à 1000 ml), parcourue par un courant de carbogène et maintenue à 37°C. On relie la préparation à un microcapteur de force et on enregistre la réponse contractile après amplification sur un enregistreur. On applique une tension de 2g à la préparation. On maintient la préparation durant 60 minutes dans la solution de Krebs-bicarbonate modifiée puis on provoque des contractions en remplaçant la solution de Krebs-bicarbonate par une solution de Krebs-potassique (NaCl 17 m M; KCl 100 m M; $NaHCO_3$ 25 m M; $KH_2PO_4$ 1 m M; $MgSO_4$ 1,2 m M; $CaCl_2$ 2,5 m M; glucose 11,5 m M; eau distillée jusqu'à 1000 ml). Lorsque la réponse contractile de la préparation est devenue reproductible on introduit, dans le bain, une quantité égale à $10^{-6}$ mole d'un composé de l'invention. Soixante minutes plus tard un nouveau spasme est provoqué par la dépolarisation potassique.

Les résultats obtenus sur l'aorte éprouvée sont alors exprimés en % de l'effet contracturant maximal avant l'incubation avec la substance à tester. A titre d'exemples, les résultats suivants ont été obtenus, les composés de formule I étant sous la forme de chlorhydrate.

| R | $R_1$ | $R_2$ | n | % de l'effet contracturant maximal |
|---|---|---|---|---|
| $C_2H_5$ | $CH_2$-C-$(CH_3)_3$ | H | 1 | 29,1 |
| $C_2H_5$ | C-$(CH_3)_3$ | H | 2 | 24,5 |
| $C_2H_5$ | $CH_2$-C-$(CH_3)_3$ | H | 2 | 25,7 |
| n-$C_3H_7$ | C-$(CH_3)_3$ | H | 1 | 43,3 |
| n-$C_3H_7$ | $CH_2$-C-$(CH_3)_3$ | H | 1 | 40,6 |
| n-$C_3H_7$ | C-$(CH_3)_3$ | H | 2 | 28,5 |
| n-$C_3H_7$ | $CH_2$-C-$(CH_3)_3$ | H | 2 | 19,8 |
| iso-$C_3H_7$ | C-$(CH_3)_3$ | H | 1 | 34,0 |
| iso-$C_3H_7$ | C-$(CH_3)_3$ | H | 2 | 7,3 |
| iso-$C_3H_7$ | $CH_2$-C-$(CH_3)_3$ | H | 2 | 21,4 |
| iso-$C_3H_7$ | C-$(CH_3)_3$ | Cl | 2 | 19,1 |
| iso-$C_3H_7$ | $CH_2$-C-$(CH_3)_3$ | Cl | 2 | 24,5 |
| n-$C_4H_9$ | C-$(CH_3)_3$ | H | 1 | 34,8 |
| n-$C_4H_9$ | $CH_2$-C-$(CH_3)_3$ | H | 1 | 29,4 |
| n-$C_4H_9$ | C-$(CH_3)_3$ | H | 2 | 34,0 |
| n-$C_4H_9$ | $CH_2$-C-$(CH_3)_3$ | H | 2 | 31,3 |

A titre de comparaison on a obtenu les résultats ci-dessous avec des composés connus:

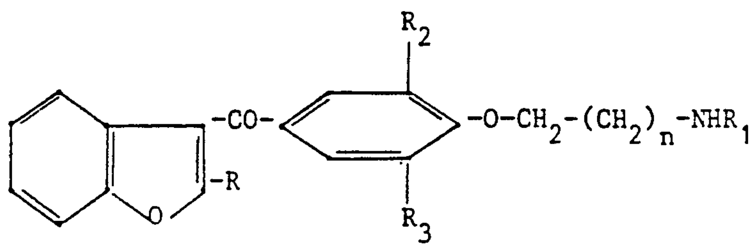

| R | $R_1$ | $R_2$ | $R_3$ | n | % de l'effet contracturant maximal |
|---|---|---|---|---|---|
| n-$C_4H_9$ | $C_2H_5$ | H | H | 2 | 80,6 |
| n-$C_4H_9$ | n-$C_3H_7$ | H | H | 2 | 73,4 |
| n-$C_4H_9$ | n-$C_4H_9$ | H | H | 2 | 58,5 |
| n-$C_4H_9$ | $C_2H_5$ | I | I | 1 | 85,1 |

Ces résultats montrent que la plupart des composés de l'invention sont plus actifs que les composés connus comme agents inhibiteurs calciques.

**II. Propriétés antiadrénergiques**

Le but de ce test est de déterminer la capacité des composés de l'invention de réduire l'augmentation de la pression sanguine induite par l'épinéphrine (effet anti-α) et l'accélération de la fréquence cardiaque induite par l'épinéphrine (effet anti-β) chez le chien préalablement anesthésié au pentobarbital et atropiné.

- Effet anti-α

On détermine d'abord pour chaque chien, la dose d'épinéphrine (entre 5 et 10 µg/kg) qui provoque une augmentation reproductible de la pression artérielle d'environ 100 mm Hg.

On administre ensuite la dose d'épinéphrine ainsi déterminée depuis 10 mg/kg par voie intraveineuse du composé à étudier. On enregistre alors le pourcentage de réduction de l'hypertension provoquée par le composé à étudier comparativement à l'hypertension obtenue précédemment (environ 100 mm Hg).

- Effet anti-β

Durant le même test que celui décrit précédemment, l'épinéphrine provoque une augmentation reproductible de la fréquence cardiaque d'environ 70 battements/min. On enregistre alors le pourcentage de

réduction de l'accélération de la fréquence cardiaque provoquée par le composé à étudier comparativement à la tachycardie mesurée précédemment (environ 70 battements). Dans les deux cas, on a exprimé les résultats de la réduction de la pression artérielle ou de la fréquence cardiaque comme suit:

(+) pour une réduction quasi nulle
\+ pour une réduction < 50 %
\+ + pour une réduction ⩾ 50 %
\+ + + pour une réduction sub-totale (réduction presque complète)

On a enregistré les résultats suivants:

| R | $R_1$ | $R_2$ | n | Effet anti-α | Effet anti-β |
|---|---|---|---|---|---|
| $C_2H_5$ | $C-(CH_3)_3$ | H | 2 | + + | + + |
| $C_2H_5$ | $CH_2-C-(CH_3)_3$ | H | 2 | + + | + + |
| $n-C_3H_7$ | $CH_2-C-(CH_3)_3$ | H | 1 | + + | + + |
| $n-C_3H_7$ | $C-(CH_3)_3$ | H | 2 | + + | + + |
| $n-C_3H_7$ | $CH_2-C-(CH_3)_3$ | H | 2 | + + | + + |
| $iso-C_3H_7$ | $C-(CH_3)_3$ | Cl | 2 | + + + | + + + |
| $iso-C_3H_7$ | $CH_2-C-(CH_3)_3$ | H | 2 | + + | + + |
| $n-C_4H_9$ | $CH_2-C-(CH_3)_3$ | H | 1 | + + + | + + + |
| $n-C_4H_9$ | $C-(CH_3)_3$ | H | 2 | + + + | + + + |
| $n-C_4H_9$ | $CH_2-C-(CH_3)_3$ | H | 2 | + + + | + + + |

A titre de comparaison, on a effectué des tests similaires avec des composes connus:

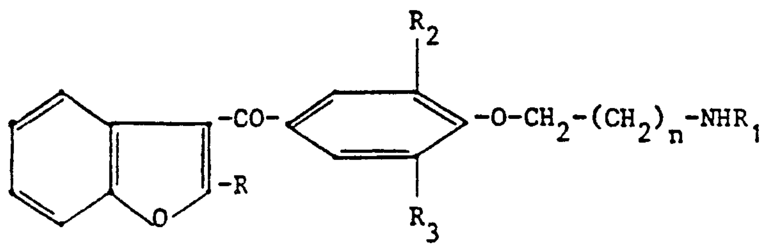

| R | $R_1$ | $R_2$ | $R_3$ | n | Effet anti-α | Effet anti-β |
|---|---|---|---|---|---|---|
| $n-C_4H_9$ | $C_2H_5$ | H | H | 2 | (+) | (+) |
| $n-C_4H_9$ | $n-C_3H_7$ | H | H | 2 | + | + |
| $n-C_4H_9$ | $n-C_4H_9$ | H | H | 2 | (+) | + |
| $n-C_4H_9$ | $C_2H_5$ | I | I | I | (+) | (+) |

Ces résultats prouvent que les composés de l'invention présentent une activité antiadrénergique α et β beaucoup plus importante que celle des composés de l'art antérieur.

Les compositions thérapeutiques selon l'invention peuvent être présentées sous toute forme convenant à l'administration en thérapie humaine ou vétérinaire. Pour ce qui concerne l'unité d'administration, celle-ci peut prendre la forme, par exemple, d'un comprimé, d'une dragée, d'une capsule, d'une gélule, d'une poudre, d'une suspension ou d'un sirop pour l'administration, orale, d'un suppositoire pour l'administration rectale ou d'une solution ou suspension pour l'administration parentérale.

Les compositions thérapeutiques de l'invention pourront comprendre, par unité d'administration, par exemple de 50 à 500 mg en poids d'ingrédient actif pour l'administration orale, de 50 à 200 mg d'ingrédient actif pour l'administration rectale et de 50 à 150 mg d'ingrédient actif pour l'administration parentérale.

Suivant la voie d'administration choisie, les compositions thérapeutiques ou vétérinaires de l'invention seront préparées en associant au moins un des composés de formule I ou un sel d'addition non toxique de ce composé avec un excipient approprié, ce dernier pouvant être constitué par exemple d'au moins un ingrédient sélectionné parmi les substances suivantes: lactose, amidons, talc, stéarate de magnésium, polyvinylpyrrolidone, acide alginique, silice colloïdale, eau distillée, alcool benzylique ou agents édulcorants.

Les exemples, non limitatifs, suivants illustrent l'invention:

**Exemple 1**

Préparation du chlorhydrate de n-butyl-2[(tertiobutylamino-2 éthoxy)-4 benzoyl]-3 benzofuranne.

a) n-Butyl-2[(bromo-2 éthoxy)-4 benzoyl]-3 benzofuranne

On agite et reflue durant 30 minutes un mélange de 93,5 g (0,25 mole) de n-butyl-2(hydroxy-4 benzoyl)-3 benzofuranne, 69 g (0,5 mole) de carbonate de potassium sec et finement broyé et 500 ml de méthyl éthyl cétone. On laisse refroidir vers 50°C et on ajoute, en une seule fois, 187,8 g (1 mole) de dibromo-1,2 éthane. On continue l'agitation et le reflux durant 18h et on ajoute 47g de dibromo-1,2 éthane supplémentaires. On continue le reflux durant 6h en suivant le progrès de la réaction par chromatographie sur couche mince (CCM). On filtre et évapore à sec sous vide. On reprend le résidu avec de l'éther éthylique, on lave à l'eau la solution éthérée puis avec une solution aqueuse d'hydroxyde de sodium à 10 % et de nouveau à l'eau. On sèche sur sulfate de sodium et on évapore à sec sous vide. On obtient ainsi 56,5 g de n-butyl-2[(bromo-2 éthoxy-4 benzoyl]-3 benzofuranne sous forme d'une huile.

Rendement : 60 %

P.F. : 46-47°C (isopropanol)

En suivant le procédé décrit ci-dessus au départ des produits appropriés et en procédant selon la nécessité à une purification chromatographique sur colonne de silice et éventuellement à une cristallisation dans un solvant approprié, on a obtenu les composés ci-dessous:

Isopropyl-2[(bromo-2 éthoxy)-4 benzoyl]-3 benzofuranne
Rendement : 37 %
Chromatographie.P.F. : 102-103°C (éther de pétrole 80-100°C).

Isopropyl-2[(bromo-3 propoxy]-4 benzoyl]-3 benzofuranne
Rendement : 49 %
Chromatographie. Produit huileux.

De même, on a préparé les composés ci-dessous selon le même procédé mais en procédant dans le diméthylformamide, à température ambiante ou, selon la nécessité, en chauffant à 50-90°C.

Ethyl-2[(bromo-3 propoxy)-4 benzoyl]-3 benzofuranne
Rendement : 80 %
Chromatographie. Huile épaisse.

n-Propyl-2[(bromo-2 éthoxy)-4 benzoyl]-3 benzofuranne
Rendement : 50 %
Chromatographie. P.F. : 68-70°C (n-hexane)

n-Propyl-2[(bromo-3 propoxy)-4 benzoyl]-3 benzofuranne
Rendement : 49 %
Chromatographie. Produit huileux.

Isopropyl-2[chloro-3(bromo-3 propoxy)-4 benzoyl]-3 benzofuranne
Rendement : 69 %
Chromatographie. Produit huileux.

n-Butyl-2[(bromo-3 propoxy)-4 benzoyl]-3 benzofuranne
Rendement : 80 %
Chromatographie. Produit huileux.

n-Butyl-2[bromo-3(bromo-2 éthoxy)-4 benzoyl]-3 benzofuranne
Rendement (brut) : 95 %
Produit huileux.

n-Butyl-2[chloro-3(bromo-2 éthoxy)-4 benzoyl]-3 benzofuranne
Rendement (brut) : 94 %
Produit huileux.

n-Butyl-2[chloro-3(bromo-3 propoxy)-4 benzoyl]-3 benzofuranne
Rendement (brut) : 89 %
Produit huileux.

b) Chlorhydrate de n-butyl-2[(tertiobutylamino-2 éthoxy)-4 benzoyl]-3 benzofuranne

On abandonne à la température de 50-80°C durant 20 à 24 heures, un mélange de 13,2 g (0,0354 mole) de n-butyl-2[(bromo-2 éthoxy)-4 benzoyl]-3 benzofuranne dissous dans 100 ml de benzène et de 26 g (0,355 mole) de tertiobutylamine dissous dans 60 ml de benzène tout en suivant l'avancement de la réaction par CCM. On évapore à sec sous vide et on reprend le résidu avec de l'éther éthylique et avec de l'eau additionnée de quelques ml d'une solution aqueuse à 10 % d'hydroxyde de sodium. On extrait à l'éther, on lave convenablement les extraits à l'eau puis on les sèche sur sulfate de sodium anhydre. On évapore à sec sous vide, on reprend le résidu avec de l'éther sec puis on acidifie avec une solution éthérée d'acide chlorhydrique.

Le chlorhydrate ainsi formé est ensuite cristallisé dans la méthyl éthyl cétone et recristallisé dans l'isopropanol.

On obtient ainsi 5,3 g de chlorhydrate de n-butyl-2[(tertiobutylamino-2 éthoxy)-4 benzoyl]-3 benzofuranne.

Rendement : 35 %

P.F. : 160-162°C.

En suivant le même procédé que ci-dessus mais au départ des produits appropriés et en chauffant le mélange réactionnel vers 50-80° C, éventuellement en vase clos, on a préparé les composés suivants:

Chlorhydrate de n-propyl-2[(tertiobutylamino-2 éthoxy)-4 benzoyl]-3 benzofuranne
Rendement : 73 %
P.F. : 164-166° C (acétate d'éthyle/méthanol)

Chlorhydrate de n-propyl-2[(néopentylamino-3 propoxy)-4 benzoyl]-3 benzofuranne
Rendement : 26 %
P.F. : 139-140° C (méthyl éthyl cétone)

Chlorhydrate d'isopropyl-2[(tertiobutylamino-2 éthoxy)-4 benzoyl]-3 benzofuranne
Rendement : 88 %
P.F. : 198-201° C (isopropanol)

Chlorhydrate d'isopropyl-2[(tertiobutylamino-3 propoxy)-4 benzoyl]-3 benzofuranne
Rendement : 41 %
P.F. : 159-162° C (cyclohexane/acétate d'éthyle)

En suivant le même procédé que ci-dessus mais au départ des produits appropriés et en procédant en milieu éthanol à une temperature comprise entre 50-60° C durant 16-20 heures, éventuellement en vase clos, on a préparé les composés suivants:

Chlorhydrate d'éthyl-2[(tertiobutylamino-3 propoxy)-4 benzoyl]-3 benzofuranne
Rendement : 53 %
P.F. : 139-142° C (acétate d'éthyle)

Chlorhydrate d'éthyl-2[(néopentylamino-3 propoxy)-4 benzoyl]-3 benzofuranne
Rendement : 10 %
P.F. : 125-128° C (acétate d'éthyle)

Chlorhydrate de n-propyl-2[(néopentylamino-2 éthoxy)-4 benzoyl]-3 benzofuranne
Rendement : 87 %
P.F. : 150-151° C (acétate d'éthyle)

Chlorhydrate d'isopropyl-2[(néopentylamino-2 éthoxy)-4 benzoyl]-3 benzofuranne
Rendement : 51 %
P.F. : 146-149° C (méthyl éthyl cétone)

Chlorhydrate d'isopropyl-2[(néopentylamino-3 propoxy)-4 benzoyl]-3 benzofuranne
Rendement : 56 %
P.F. : 153-156° C (isopropanol)

Chlorhydrate d'isopropyl-2 chloro-3[(tertiobutylamino-3 propoxy)-4 benzoyl]-3 benzofuranne
Rendement : 84 %
P.F. : 148-150° C (acétate d'éthyle)

Chlorhydrate d'isopropyl-2[chloro-3(néopentylamino-3 propoxy)-4 benzoyl]-3 benzofuranne
Rendement : 44 %
P.F. : 138-141° C (acétate d'éthyle)

Chlorhydrate de n-butyl-2[(néopentylamino-2 éthoxy)-4 benzoyl]-3 benzofuranne
Rendement : 45 %
P.F. : 152-154° C (eau)

Chlorhydrate de n-butyl-2[(tertiobutylamino-3 propoxy)-4 benzoyl]-3 benzofuranne
Rendement : 33 %
P.F. : 142-144° C (isopropanol)

Chlorhydrate de n-butyl-2[(néopentylamino-3 propoxy)-4 benzoyl]-3 benzofuranne
Rendement : 39 %
P.F. : 140-142° C (eau)

**Exemple 2**

Préparation du chlorhydrate d'éthyl-2[(tertiobutylamino-2 éthoxy)-4 benzoyl]-3 benzofuranne

a) Ethyl-2 [(hydroxy-2 éthoxy)-4 benzoyl]-3 benzofuranne

On agite à la température ambiante durant 30 minutes une suspension de 51,7 g (0,379 mole) de carbonate de potassium sec et finement pulvérisé dans une solution de 49,9 g (0,18745 mole) d'éthyl-2 (hydroxy-4 benzoyl)-3 benzofuranne dans 400 ml de diméthylformamide puis on chauffe vers 50° C durant 20 minutes. Après refroidissement, on ajoute, en une fois, 118 g (0,937 mole) de bromo-2 éthanol. On agite et on chauffe vers 60-70° C durant 24 heures puis on laisse refroidir et on verse dans de l'eau froide. On extrait à l'éther, on lave à l'eau, avec une solution aqueuse d'hydroxyde de sodium à 10 % et de nouveau à l'eau.

On sèche sur sulfate de sodium et on évapore sous vide. On reprend le résidu huileux dans l'éther de pétrole 40-60° C, on agite et on filtre.

On obtient ainsi 56,7 g d'éthyl-2[(hydroxy-2 éthoxy)-4 benzoyl]-3 benzofuranne.

Rendement : 97,4 %

P.F. : 78-83°C
En suivant le même procédé que décrit ci-dessus mais au départ des produits appropriés on a préparé le n-butyl-2[chloro-3(hydroxy-2 éthoxy)-4 benzoyl]-3 benzofuranne (produit huileux)
Rendement : 97 %

b) Ethyl-2[(p-toluènesulfonyloxy-2 éthoxy)-4 benzoyl]-3 benzofuranne
Sous agitation et en maintenant la température en dessous de 20°C, on ajoute par petites fractions 14 g (0,0734 mole) de chlorure de p-toluènesulfonyle à 20 g (0,0644 mole) d'éthyl-2[(hydroxy-2 éthoxy)-4 benzoyl]-3 benzofuranne dans 21 ml de pyridine. On agite à la température ambiante durant 4h et on verse le milieu réactionnel dans 120 ml d'eau glacée contenant 35 ml d'acide chlorhydrique concentré. On extrait au benzène, on lave soigneusement à l'eau, on sèche sur sulfate de sodium et on évapore à sec sous vide. On reprend le résidu encore chaud avec de l'éther de pétrole 40-60°C, on agite et on essore.
On obtient ainsi 26 g d'éthyl-2[(p-toluènesulfonyloxy-2 éthoxy)-4 benzoyl]-3 benzofuranne.
Rendement : 86,9 %
P.F. : 95-98°C.
En suivant le même procédé que décrit précédemment mais au départ des produits appropriés on a préparé le n-butyl-2 chloro-3[(p-toluènesulfonyloxy-2 éthoxy)-4 benzoyl]-3 benzofuranne (produit huileux)
Rendement : 100 %

c) Chlorhydrate d'éthyl-2[(tertiobutilamino-2 éthoxy)-4 benzoyl]-3 benzofuranne
On chauffe à 50-70°C durant 20h, dans un vase clos, une solution de 13 g (0,028 mole) d'éthyl-2[(p-toluènesulfonyloxy-2 éthoxy)-4 benzoyl]-3 benzofuranne et de 22 g (0,29 mole) de tertiobutylamine dans 100 ml de benzène tout en suivant l'évolution de la réaction par CCM. On évapore à sec, en reprend le résidu avec de l'éther éthylique, de l'eau additionnée de quelques ml d'une solution aqueuse d'hydroxyde de sodium à 10 % puis avec de l'eau. On sèche sur sulfate de sodium, on forme le chlorhydrate et on recristallise dans l'isopropanol.
On obtient ainsi 6,6 g de chlorhydrate d'éthyl-2[(tertiobutylamino-2 éthoxy-4 benzoyl]-3 benzofuranne
Rendement : 59 %
P.F. : 173-175°C
De la même manière mais au départ des produits appropriés, on a préparé le chlorhydrate d'éthyl-2[(néopentylamino-2 éthoxy-4 benzoyl]-3 benzofuranne
Rendement : 42 %
P.F. : 138-139°C.

**Exemple 3**

Préparation du n-propyl-2[(tertiobutylamino-3 propoxy)-4 benzoyl]-3 benzofuranne base et de son chlorhydrate.

a) Base libre
On chauffe à 50-80°C durant 20 à 24 heures un mélange de 14,2 g (0,0354 mole) de n-propyl-2[(bromo-3 propoxy)-4 benzoyl]-3 benzofuranne dissous dans 100 ml de benzène et de 26 g (0,355 mole) de tertiobutylamine dissous dans 60 ml de benzène tout en suivant l'avancement de la réaction par CCM.
On évapore à sec sous vide et on reprend le résidu avec de l'éther éthylique et avec de l'eau additionnée de quelques ml d'une solution aqueuse à 10 % d'hydroxyde de sodium. On extrait à l'éther, on lave convenablement les extraits à l'eau, on les sèche sur sulfate de sodium anhydre et on évapore à sec sous vide.
On obtient ainsi le n-propyl-2[tertiobutylamino-3 propoxy)-4 benzoyl]-3 benzofuranne sous forme de base libre (produit huileux).
Spectre R.M.N. ($CDCl_3$, référence : tétraméthylsilane)

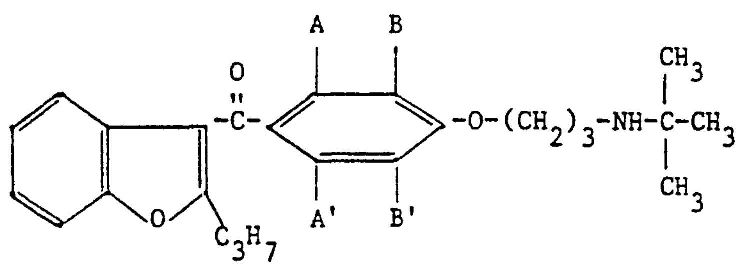

Déplacements chimiques
$\delta_H$AA' : 7,73 ppm
$\delta_H$ aromatique : 7,13 ppm (massif)
$\delta_H$BB' : 6,84 pm

$\delta_H$<u>OCH₂</u> : 4,07 ppm (triplet)

$\delta$ H ( aromatique $CH_2$ ) : 2,83 ppm (massif)
( $NCH_2$ )
$\delta$ H ( $OCH_2$ - <u>$CH_2$</u> ) : 1,85 ppm (massif)
( <u>$CH_2$</u>, $CH_3$ )

$\delta_H NH(CH_3)_3$ : 1,08 ppm
$\delta_H$ $CH_2$-<u>$CH_3$</u> : 0,91 ppm (triplet)

<u>Constante de couplage</u>
$J_{A,B}$ = 8,5 cps

b) <u>Chlorhydrate</u>
On reprend la base obtenue ci-dessus avec de l'éther éthylique sec, on acidifie avec une solution éthérée d'acide chlorhydrique et on recristallise dans l'acétate d'éthyle.
On obtient ainsi 5,6 g de chlorhydrate de n-propyl-2[(tertiobutylamino-3 propoxy)-4 benzoyl]-3 benzofuranne.
Rendement : 37 %
P.F. : 133-135°C

**Exemple 4**

Suivant des techniques pharmaceutiques connues, on a préparé une capsule contenant les ingrédients suivants:

| <u>Ingrédient</u> | <u>mg</u> |
|---|---|
| Composé de l'invention | 100,0 |
| Amidons | 99,5 |
| Silice colloïdale | 0,5 |
| | 200,0 |

**Revendications**

1. Dérivés de benzofuranne correspondant à la formule générale:

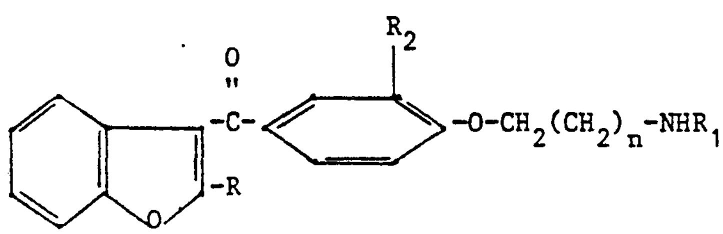

ainsi que leurs sels d'addition non toxiques, dans laquelle:
R représente un radical éthyle, n-propyle, isopropyle ou n-butyle
$R_1$ représente un radical alkyle ramifié ayant de 4 à 7 atomes de carbone dans lequel au moins un des atomes de carbone ne porte pas d'hydrogène
$R_2$ représente hydrogène, chlore ou brome
n représente 1 ou 2.

2. Dérivés de benzofuranne selon la revendication 1 dans laquelle $R_1$ représente un radical tertiobutyle ou néopentyle.

3. n-Butyl-2[(néopentylamino-2 éthoxy)-4 benzoyl]-3 benzofuranne et ses sels d'addition non toxiques.

4. Isopropyl-2[chloro-3(tertiobutylamino-3 propoxy)-4 benzoyl]-3 benzofuranne et ses sels d'addition non toxiques.

5. Isopropyl-2[chloro-3(néopentylamino-3 propoxy-4 benzoyl]-3 benzofuranne et ses sels d'addition non toxiques.

6. Isopropyl-2[(néopentylamino-3 propoxy)-4 benzoyl]-3 benzofuranne et ses sels d'addition non toxiques.

7. n-Propyl-2[(néopentylamino-3 propoxy)-4 benzoyl]-3 benzofuranne et ses sels d'addition non toxiques.

8. Dérivés de benzofuranne selon l'une quelconque des Revendications 1 à 7 caractérisés en ce que le sel d'addition non toxique est le chlorhydrate.

9. Procédé de préparation de dérivés de benzofuranne selon la Revendication 1 caractérisé en ce que l'on condense, dans un milieu inerte et à une température comprise entre la température ambiante et 80°C, un composé benzofurannique de formule générale:

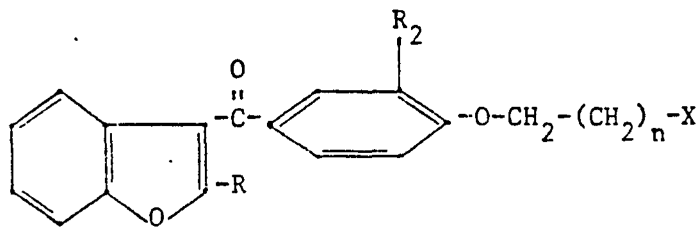

dans laquelle R, $R_2$ et n ont la même signification que dans la Revendication 1 et X représente un atome d'halogène ou un groupement p-toluènesulfonyloxy, avec une amine de formule générale:

$H_2N\text{-}R_1$

dans laquelle $R_1$ a la même signification que dans la Revendication 1 pour former le dérivé souhaité de benzofuranne que l'on fait réagir, si on le désire, avec un acide organique ou inorganique approprié pour obtenir un sel d'addition non toxique.

10. Composition pharmaceutique ou vétérinaire caractérisée en ce qu'elle comprend, comme principe actif, au moins un dérivé de benzofuranne selon la Revendication 1, en association avec un véhicule pharmaceutique ou un excipient approprié.

11. Composition pharmaceutique ou vétérinaire caractérisée en ce qu'elle comprend, comme principe actif, au moins un dérivé de benzofuranne selon la Revendication 2 en association avec un véhicule pharmaceutique ou un excipient approprié.

12. Composition pharmaceutique ou vétérinaire caractérisée en ce qu'elle comprend, comme principe actif au moins un dérivé de benzofuranne selon l'une quelconque des Revendications 4 à 7, en association avec un véhicule pharmaceutique ou un excipient approprié.

**Patentansprüche**

1. Benzofuranderivate entsprechend der allgemeinen Formel

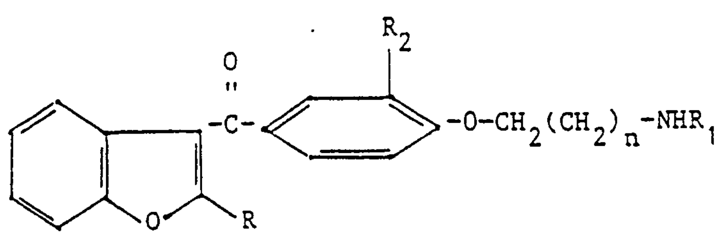

und die pharmazeutisch annehmbaren Säure-Additionssalze derselben, worin

R ein Ethyl-, n-Propyl-, Isopropyl- oder n-Butylrest ist;

$R_1$ einen verzweigtkettigen Alkylrest mit 4 bis 7 Kohlenstoffatomen bedeutet, worin mindestens eines der Kohlenstoffatome kein Wasserstoffatom aufweist;

$R_2$ Wasserstoff, Chlor oder Brom bedeutet;

n = 1 oder 2 ist.

2. Benzofuranderivate nach Anspruch 1, worin $R_1$ einen t-Butyl- oder Neopentylrest bedeutet.

3. 2-n-Butyl-3-[4-(2-neopentylamino-ethoxy)-benzoyl]-benzofuran und die pharmazeutisch annehmbaren Säure-Additionssalze desselben.

4. 2-Isopropyl-3-[3-chlor-4-(3-t-butylamino-propoxy)-benzoyl]-benzofuran und die pharmazeutisch annehmbaren Säure-Additionssalze desselben.

5. 2-Isopropyl-3-[3-chlor-4-(3-neopentylamino-propoxy)-benzoyl]-benzofuran und die pharmazeutisch annehmbaren Säure-Additionssalze desselben.

6. 2-Isopropyl-3-[4-(3-neopentylamino-propoxy)-benzoyl]-benzofuran und die pharmazeutisch annehmbaren Säure-Additionssalze desselben.

7. 2-n-Propyl-3-[4-(3-neopentylamino-propoxy)-benzoyl]-benzofuran und die pharmazeutisch annehmbaren Säure-Additionssalze desselben.

8. Benzofuranderivat nach irgendeinem der Ansprüche 1 bis 7, worin das pharmazeutisch annehmbare Säure-Additionssalz das Hydrochlorid ist.

9. Ein Verfahren zur Herstellung der Benzofuranderivate nach Anspruch 1, das umfaßt: das Kondensieren einer Benzofuranverbindung der allgemeinen Formel

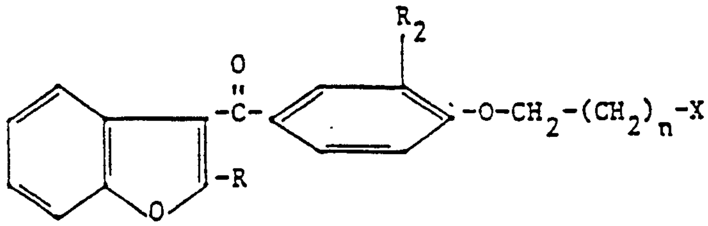

in welcher R, $R_2$ und n dieselbe Bedeutung wie in Anspruch 1 haben und X ein Halogenatom oder die p-Toluolsulfonyloxygruppe bedeutet, in einem inerten Medium und bei einer Temperatur zwischen Raumtemperatur und 80°C mit einem Amin der allgemeinen Formel

$H_2N-R_1$

in welcher $R_1$ dieselbe Bedeutung wie in Anspruch 1 hat, unter Bildung des gewünschten Benzofuranderivates, das gegebenenfalls mit einer entsprechenden organischen oder anorganischen SSäure unter Bildung eines pharmazeutisch annehmbaren Säure-Additionssalzes umgesetzt wird.

10. Eine pharmazeutische oder veterinärmedizinische Zusammensetzung, enthaltend als wesentlichen aktiven Bestandteil mindestens ein Benzofuranderivat nach Anspruch 1 in Verbindung mit einem pharmazeutischen Träger oder Hilfsmittel.

11. Eine pharmazeutische oder veterinärmedizinische Zusammensetzung, enthaltend als wesentlichen aktiven Bestandteil mindestens ein Benzofuranderivat nach Anspruch 2 in Verbindung mit einem pharmazeutischen Träger oder Hilfsmittel.

12. Eine pharmazeutische oder veterinärmedizinische Zusammensetzung, enthaltend als wesentlichen aktiven Bestandteil mindestens ein Benzofuranderivat nach irgendeinem der Ansprüche 4 bis 7 in Verbindung mit einem pharmazeutischen Träger oder Hilfsmittel.

**Claims**

1. Benzofuran derivatives corresponding to the general formula:

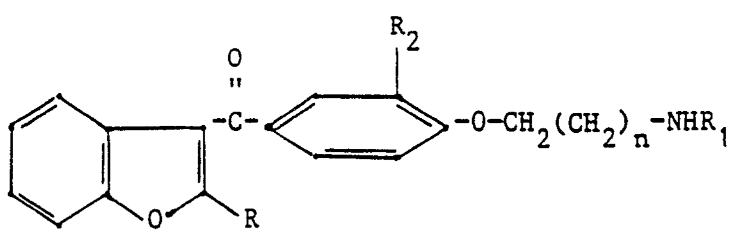

and the pharmaceutically acceptable acid addition salts thereof, wherein:

R represents an ethyl, n-propyl, isopropyl or n-butyl radical;

$R_1$ represents a branched-chain alkyl radical having from 4 to 7 carbon atoms wherein at least one of the carbon atoms does not bear a hydrogen atom;

$R_2$ represents hydrogen, chlorine or bromine ;

n is 1 or 2.

2. Benzofuran derivatives as claimed in Claim 1 wherein $R_1$ represents a tert-butyl or neopentyl radical.

3. 2-n-Butyl-3-[4-(2-neopentylamino-ethoxy)-benzoyl]-benzofuran and the pharmaceutically acceptable acid addition salts thereof.

4. 2-Isopropyl-3-[3-chloro-4-(3-tert-butylamino-propoxy)-benzoyl]-benzofuran and the pharmaceutically acceptable acid addition salts thereof.

5. 2-Isopropyl-3-[3-chloro-4-(3-neopentylamino-propoxy)-benzoyl]-benzofuran and the pharmaceutically acceptable acid addition salts thereof.

6. 2-Isopropyl-3-[4-(3-neopentylamino-propoxy)-benzoyl]-benzofuran and the pharmaceutically acceptable acid addition salts thereof.

7. 2-n-Propyl-3-(4-(3-neopentylamino-propoxy)-benzoyl]-benzofuran and the pharmaceutically acceptable acid addition salts thereof.

8. Benzofuran derivatives as claimed in any one of Claims 1 to 7 wherein the pharmaceutically acceptable acid addition salt is the hydrochloride.

9. A process for the preparation of benzofuran derivatives as claimed in Claim 1 which process comprises condensing in an inert medium and at a temperature between room-temperature and 80°C, a benzofuran compound of the general formula:

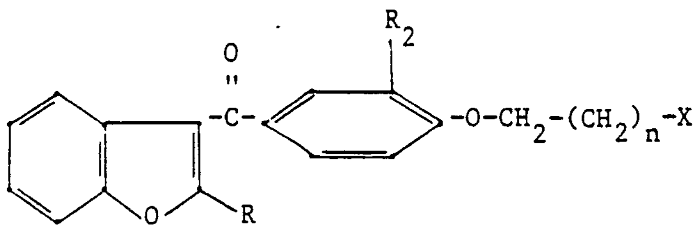

in which R, $R_2$ and n have the same meanings as in Claim 1 and X represents a halogen atom or the p-toluenesulphonyloxy group, with an amine of the general formula:

$H_2N-R_1$

in which $R_1$ has the same meaning as in Claim 1, to form the required benzofuran derivative which, if desired, is reacted with an appropriate organic or inorganic acid to produce a pharmaceutically acceptable acid addition salt.

10. A pharmaceutical or veterinary composition containing as essential active ingredient at least one benzofuran derivative as claimed in Claim 1 in association with a pharmaceutical carrier or excipient therefor.

11. A pharmaceutical or veterinary composition containing as essential active ingredient at least one benzofuran derivative as claimed in Claim 2 in association with a pharmaceutical carrier or excipient therefor.

12. A pharmaceutical or veterinary composition containing as essential active ingredient, at least one benzofuran derivative as claimed in any one of Claims 4 to 7, in association with a pharmaceutical carrier or excipient therefor.